# EUROPEAN PATENT APPLICATION

(11) **EP 3 858 976 A1**
(43) Date of publication of application: **04.08.2021**
(21) Application number: 18929747.6
(22) Date of filing: 09.08.2018
(51) Int. Cl.: C12N 1/20, C12R 1/63, C02F 3/34

(54) **BACTERIAL STRAIN HAVING VERY STRONG SULFATE REDUCTION ABILITY AND USE THEREOF**

(71) Applicant: China University of Petroleum-Beijing, Beijing 102249 (CN)
(72) Inventor: WAN, Yunyang, Beijing 102249 (CN); LUO, Na, Beijing 102249 (CN); XIAN, Chenggang, Beijing 102249 (CN)
(74) Representative: reuteler & cie SA
(86) International application number: PCT/CN2018/099491
(87) International publication number: WO 2020/029149

(57) **Abstract**

Provided is a *Cupidesulfovibrio* sp. XJ01 strain which has a sulfate reduction ability, the deposit number thereof being CGMCC No.16138. The strain can remove no less than 90% of 0.5-1 g/L of SO₄²⁻ in one day, and the maximum content of SO₄²⁻ removed in five days is 50 g/L. The strain can be used for the rapid reduction of sulfates.

SEQUENCE LIST

<110>  CHINA UNIVERSITY OF PETROLEUM-BEIJING

<120>  BACTERIAL STRAIN HAVING VERY STRONG SULFATE REDUCTION ABILITY AND USE
THEREOF

<130>  WPI18CN0267X

<160>  1

<170>  PatentIn version 3.5

<210>  1
<211>  1445
<212>  DNA
<213>  Cupidesulfovibrio sp.

<400>  1
tatctgagct gcgtctaccg attctgcgtt caacggtaca ggcctgagta aagcggcgca      60

cgggtgagta acgcgtggat gatctgccca tgagttggga ataacggctg gaaacggtcg     120

ctaataccga atacgctccg atttcacatt tcgggggaaa ggtggcctct gcttgcaagc     180

taccgctcat ggatgagtcc gcgtcccatt agctagttgg cggggtaatg gcccaccaag     240

gcgacgatgg gtagccgacc tgagagggtg atcggccaca ctgggactgg aacacggccc     300

agactcctac gggaggcagc agtggggaat attgcgcaat gggcgaaagc ctgacgcagc     360

gacgccgcgt gagggatgaa ggccttcggg tcgtaaacct ctgtcaggag ggaagaaccg     420

ccatggtgct aatcagccat ggtctgacgg tacctccaaa ggaagcaccg gctaactccg     480

tgccagcagc cgcggtaata cggagggtgc aagcgttaat cggaatcact gggcgtaaag     540

cgcacgtagg ctgtttggta agtcaggggt gaaatcccgc ggctcaaccg cggaattgcc     600

cttgatactg ctggacttga gttcgggaga gggtggcgga attccaggtg taggagtgaa     660

atccgtagat atctggagga acatcagtgg cgaaggcggc cacctggacc gatactgacg     720

ctgaggtgcg aaagcgtggg gagcaaacag gattagatac cctggtagtc cacgctgtaa     780

acgatggatg ctaggtgtcg gggccttgag cttcggtgcc gcagctaacg cgttaagcat     840

cccgcctggg gagtacggtc gcaaggctga aactcaaaga aattgacggg ggcccgcaca     900

agcggtggag tatgtggttt aattcgatgc aacgcgaaga accttaccca ggcttgacat     960

ccgggaaacc ctcccgaaaa ggaggggtgc tcttcggaga atcccgagac aggtgctgca    1020

tggctgtcgt cagctcgtgc cgtgaggtgt tgggttaagt cccgcaacga gcgcaacccc    1080

tatccttagt tgctaccagg cgatgctggg cactctatgg agaccgcccc ggttaacggg    1140

gaggaaggtg gggatgacgt caagtcatca tggcccttac gcctggggct acacacgtac    1200

tacaatggcg cacacaaagg gcagcgatac cgtgaggtgg agccaatccc aaaaaatgcg    1260

tcccagtccg gattgcagtc tgcaactcga ctgcatgaag ttggaatcgc tagtaattcg    1320

agatcagcat gctcgggtga atgcgttccc gggccttgta cacaccgccc gtcacaccac 1380

gaaagtcggt tttacccgat accggtgagc caaccgcaag gaggcagccg tctacgtaga 1440

cccgg 1445

## Description

### Technical field

The present disclosure relates to a sulfate-reducing bacterial strain and use thereof, and in particular, to the *Cupidesulfovibrio* sp. strain XJ01 and use thereof. The strain showed an extraordinary sulfate-reducing ability. The present disclosure relates to the field of screening and applications of microorganisms.

### Background Art

Sulfate-reducing bacteria is a main group of microorganisms that participate in the sulfur cycle and cause the increase in sulfides such as hydrogen sulfide in oil reservoirs. Because of their strong environmental adaptability, their potential applications in the treatment of sulfate-rich wastewater, in the degradation of organic matter, and in the oil refining industry have become the research focuses of many scientists. A lot of work has been done by the domestic and foreign scientists on sulfate-reducing bacteria. According to statistics, as of 2015, among the sulfate-reducing bacteria with *desulfo-* in the name, there are already 50 genera and more than 220 species (Wan Yunyang and Zhao Guoping, 2017). However, the sulfate-reducing bacteria isolated by the domestic and foreign scientists so far generally show a low reducing ability, and typically grow at a sulfate radical concentration <9 g/L. Strains with an extraordinary sulfate-reducing ability are rarely reported.

There is still a need in the art to find a sulfate-reducing bacterium with a high reducing ability and a fast reduction rate, so that a good performance can be expected when used in the field of sulfate-rich wastewater or the like.

### Summary of Invention

An objective of the present disclosure is to provide a sulfate-reducing bacterium having a high reducing ability and a fast reduction rate.

Another objective of the present disclosure is to provide the use of the bacterium, particularly with a good performance expectable in the field of sulfate-rich wastewater or the like.

The inventors screened the wellhead produced fluid from the Liaohe Oil Field and obtained a strain capable of reducing sulfate quickly and efficiently, which is named strain XJ01 in the present disclosure. According to the morphological, physiological, and biochemical characteristics and a 16S rDNAgene sequence analysis, XJ01 may be identified as a potentially new species in *Desulfovibrionales.* In the present disclosure, this strain is also called *Cupidesulfovibrio* sp. XJ01, which was deposited in China General Microbiological Culture Collection Center (CGMCC) on July 20, 2018 (Address: Institute of Microbiology Chinese Academy of Sciences, Building 3, Yard No.1 West Beichen Road, Chaoyang District, Beijing, 100101) with the deposit number CGMCC No. 16138 and the taxonomic name *Cupidesulfovibrio* sp.

The *Cupidesulfovibrio* sp. strain XJ01 according to the present disclosure has an extraordinary sulfate-reducing ability. Experiments on the growth in media with different initial concentrations of sulfate showed that:
(1) at [SO₄²⁻]=0.5 g/L, the strain can achieve a sulfate reduction of 100% in 1 day;
(2) at [SO₄²⁻]=1 g/L, a sulfate reduction over 90% in 1 day and up to 100% in 2 days;
(3) at [SO₄²⁻]=10 g/L, a sulfate reduction of up to 60% in 2 days;
(4) at [SO₄²⁻]=20 g/L, a sulfate reduction of up to 44% in 3 days;
(5) at [SO₄²⁻]=30 g/L, a sulfate reduction of up to 37% in 5 days;
(6) at [SO₄²⁻]=40 g/L, a sulfate reduction of up to 25% in 5 days;
(7) at [SO₄²⁻]=50 g/L, a sulfate reduction of up to 13% in 5 days;
(8) at [SO₄²⁻]>50 g/L, the strain no longer grew.

Thus it can be seen that the strain has an extraordinary sulfate-reducing ability.

Accordingly, in one aspect, the present disclosure provides a sulfate-reducing bacterium (*Cupidesulfovibrio* sp.), deposited with the deposit number CGMCC No. 16138.

The sulfate-reducing bacterium (*Cupidesulfovibrio* sp.) provided by the present disclosure has a 16S rDNA sequence represented by SEQ ID No. 1.

In another aspect, the present disclosure also provides a sulfate-reducing bacterial preparation, which comprises the sulfate-reducing bacterium according to the present disclosure and may be a solid or liquid bacterial preparation.

In another aspect, the present disclosure also provides a method for culturing the sulfate-reducing bacterium or the sulfate-reducing bacterial preparation, the method comprising: culturing the sulfate-reducing bacterium or the sulfate-reducing bacterial preparation in a fermentation medium.

According to a specific embodiment of the present disclosure, in the method according to the present disclosure, the [SO₄²⁻] in the medium is less than or equal to 50 g/L, preferably 1 g/L to 50 g/L, and the most preferable [SO₄²⁻] is 10 g/L.

According to a specific embodiment of the present disclosure, in the method according to the present disclosure, the culturing conditions are: a pH of 6 to 9, and a temperature of 20 to 45°C.

In another aspect, the present disclosure also provides metabolites of a sulfate-reducing bacterium, which are produced according to the above-mentioned method for culturing the sulfate-reducing bacterium.

In another aspect, the present disclosure also provides use of the sulfate-reducing bacterium, the sulfate-reducing bacterial preparation, or the metabolites in sulfate reduction.

According to a specific embodiment of the present disclosure, the sulfate-reducing bacterium is used to treat a sulfate-containing waste liquid. In a more specific use, the [SO₄²⁻] in the waste liquid is less than or equal to 50 g/L. Preferably, the [SO₄²⁻] in the waste liquid is 0.5 g/L to 50 g/L, more preferably 1 g/L to 10 g/L.

The *Cupidesulfovibrio* sp. strain XJ01 according to the present disclosure has a high sulfate-reducing ability and a fast reduction rate, and is expected to have a good performance when used in the field of sulfate-rich wastewater or the like.

### Description of Drawings

Figure 1 is a scanning electron microscope image of the *Cupidesulfovibrio* sp. Strain XJ01 according to the present disclosure.
Figure 2 is a phylogenetic tree of the screened strain and similar strains constructed in the examples.
Figures 3A to 3B show the reducing ability of the *Cupidesulfovibrio* sp. strain XJ01 according to the present disclosure over time as assayed under different initial concentrations of sulfate.

### Deposition of Microorganisms for the Purpose of Patent Procedures

Date of deposit: July 20, 2018
Depository authority: China General Microbiological Culture Collection Center (CGMCC);
Address of depository authority: Institute of Microbiology Chinese Academy of Sciences, Building 3, Yard No.1 West Beichen Road, Chaoyang District, Beijing, 100101;
Deposit number: CGMCC No. 16138;
Taxonomic name: *Cupdesulfovibrio* sp.

### Detailed Description of Invention

In order to have a better understanding of the technical features, objectives and beneficial effects of the present disclosure, the characteristics of the *Cupdesulfovibrio* sp. strain XJ01 according to the present disclosure and the technical effects produced thereby in use will be described hereinafter, but the present invention is not limited thereto by any means.

### Example 1: Isolation and identification of the sulfate-reducing bacterial strain Cupidesulfovibrio sp. XJ01

### 1. Sampling and enrichment culturing

In an anaerobic incubator, an appropriate amount of produced fluid from the Liaohe Oil Field (41°05.538'N 121°44.050'E) was taken and inoculated into the culture medium for sulfate-reducing bacteria recommended by the American Petroleum Institute (API) (hereinafter referred to as "API medium"). The main components of the API medium are:
Solution A (g/L): sodium chloride, 10; magnesium sulfate heptahydrate, 0.01; dipotassium hydrogen phosphate, 0.01; yeast extract powder, 1.0; sodium lactate (60%), 4 mL; cysteine hydrochloride, 0.5; resazurin, 0.001; adjusted to pH 6.5 with 10 M potassium hydroxide;
Solution B (g/L): ferrous ammonium sulfate, 0.2; ascorbic acid, 0.2.

Since there were ingredients easily decomposed and oxidized at high temperatures among the reagents, different methods were required for sterilization, and the culture medium was separately formulated as Solutions A and B.

Solution A was formulated first. The reagents prescribed in Solution A were precisely weighed and dissolved completely in 1 L deionized water. Then the solution was aliquoted in anaerobic tubes (4 mL each), the plugs were tightly closed, and the tubes were ready for gas charging. The prepared medium was purged with nitrogen to remove oxygen by 6 repeats of turning on the vacuum pump and gas charging device, vacuuming the anaerobic tubes, and then charging them with nitrogen (at a partial pressure between 0.2 and 0.5). After the gas charging, the nitrogen in the anaerobic tubes that exceeded the normal pressure was released with a needle, and the tubes were sterilized at 15 pounds, 121°C for 20 minutes, and cooled for use.

Fresh Solution B was separately formulated, and injected through a 0.25 µm sterile filter into a sterilized empty anaerobic bottle as a stock solution.

After medium A was prepared, 0.2 mL Solution B was added thereto to make a complete medium. The stock solution was preserved in a 4°C refrigerator if not used.

Culturing was conducted at 35°C for 7 days. When the culture medium showed obvious black turbid, the gas above the liquid surface of the culture medium was extracted and reacted with a soaked lead acetate test paper. The test paper turned black and a strong rotten egg smell can be smelled, indicating that the sulfate-reducing bacteria had proliferated in large numbers. The above bacteria liquid was used as an inoculum to carry out further enrichment culturing 2 to 3 times.

### 2. Purification

Single colonies were obtained by the Hungate Roll-tube method from the enrichment culture from above "1. Sampling and enrichment culturing", and observed for morphology. Non-overlapping individual colonies that differ from each other in morphological features such as size, surface characteristics, edge, and color, were selected and cultured at 35°C for 1 to 2 days. The single colonies picked may be stained and observed under a microscope, and if not pure, the above steps were repeated several times until a pure strain was obtained.

### 3. Identification

After the above step "2. Purification", a strain XJ01 was obtained in the present disclosure by screening, and identified as follows.

### (1) Morphological, physiological, and biochemical identification

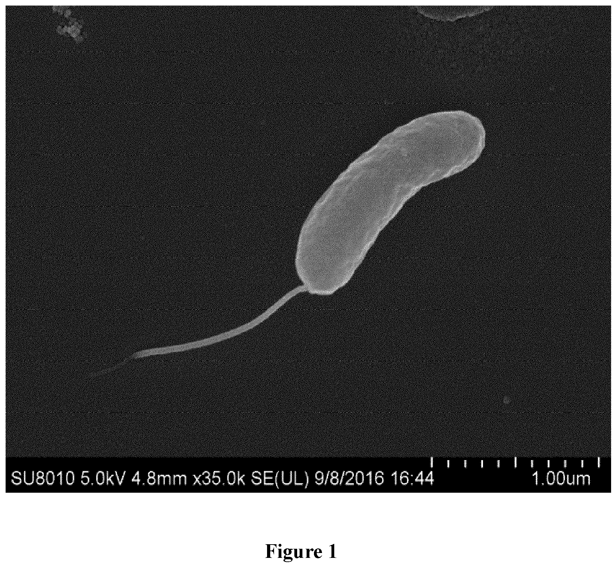

According to the "Environmental Geological Microbiology Experimental Guidance", XJ01 is a strictly anaerobic, mobile, arc-shaped gram-negative bacterium having a size of 0.4-0.6 µm ×2.5-3.7 µm and a flagellum (Figure 1). The strain grows at a pH of 6 to 9 (the optimum pH for growth is 7), at a temperature of 20-45°C (the optimum temperature for growth is 35°C), without the need of sodium chloride. It is catalase positive and oxidase negative, can utilize lactic acid, glycerol, sodium lactate, trisodium citrate and methanol as electron donors, and utilize sulfate and sulfite as electron acceptors.

### (2) Molecular biological identification

Total DNA was extracted from the bacterial culture growing in the exponential phase by using the Bacterial Deoxyribonucleic Acid (DNA) Extraction Kit (Shanghai Sangon®). With total DNA as a template, a polymerase chain reaction (PCR) amplification was carried out with universal primers 27F and 1492R (synthesized by Shanghai Sangon®) in the following reaction system: 10×buffer (containing Mg²⁺), 5 µL; 2.5 mM dNTP, 2 µL; 10 uM forward primer, 1 µL; 10 uM reverse primer, 1 µL; template, 2 µL; Taq, 0.5 µL; ddH₂O, 38.5 µL. Reaction conditions: template pre-denaturation for 5 min, denaturation at 94°C for 1 min, annealing at 55°C for 1.5 min, extension at 72°C for 1.5 min, for 35 cycles in total, with extension at 72°C for 20 min in the last cycle. The PCR product was sequenced by Shanghai Sangon, and the obtained sequence is shown by SEQ ID No. 1.

SEQ ID No.1:

TATCTGAGCTGCGTCTACCGATTCTGCGTTCAACGGTACAGGCCTGAGTAA
AGCGGCGCACGGGTGAGTAACGCGTGGATGATCTGCCCATGAGTTGGGAA
TAACGGCTGGAAACGGTCGCTAATACCGAATACGCTCCGATTTCACATTTCG

GGGGAAAGGTGGCCTCTGCTTGCAAGCTACCGCTCATGGATGAGTCCGCGT
CCCATTAGCTAGTTGGCGGGGTAATGGCCCACCAAGGCGACGATGGGTAGC
CGACCTGAGAGGGTGATCGGCCACACTGGGACTGGAACACGGCCCAGACT
CCTACGGGAGGCAGCAGTGGGGAATATTGCGCAATGGGCGAAAGCCTGAC
GCAGCGACGCCGCGTGAGGGATGAAGGCCTTCGGGTCGTAAACCTCTGTC
AGGAGGGAAGAACCGCCATGGTGCTAATCAGCCATGGTCTGACGGTACCTC
CAAAGGAAGCACCGGCTAACTCCGTGCCAGCAGCCGCGGTAATACGGAGG
GTGCAAGCGTTAATCGGAATCACTGGGCGTAAAGCGCACGTAGGCTGTTTG
GTAAGTCAGGGGTGAAATCCCGCGGCTCAACCGCGGAATTGCCCTTGATAC
TGCTGGACTTGAGTTCGGGAGAGGGTGGCGGAATTCCAGGTGTAGGAGTG
AAATCCGTAGATATCTGGAGGAACATCAGTGGCGAAGGCGGCCACCTGGA
CCGATACTGACGCTGAGGTGCGAAAGCGTGGGGAGCAAACAGGATTAGAT
ACCCTGGTAGTCCACGCTGTAAACGATGGATGCTAGGTGTCGGGGCCTTGA
GCTTCGGTGCCGCAGCTAACGCGTTAAGCATCCCGCCTGGGGAGTACGGTC
GCAAGGCTGAAACTCAAAGAAATTGACGGGGGCCCGCACAAGCGGTGGA
GTATGTGGTTTAATTCGATGCAACGCGAAGAACCTTACCCAGGCTTGACAT
CCGGGAAACCCTCCCGAAAAGGAGGGGTGCTCTTCGGAGAATCCCGAGAC
AGGTGCTGCATGGCTGTCGTCAGCTCGTGCCGTGAGGTGTTGGGTTAAGTC
CCGCAACGAGCGCAACCCCTATCCTTAGTTGCTACCAGGCGATGCTGGGCA
CTCTATGGAGACCGCCCCGGTTAACGGGGAGGAAGGTGGGGATGACGTCA
AGTCATCATGGCCCTTACGCCTGGGGCTACACACGTACTACAATGGCGCAC
ACAAAGGGCAGCGATACCGTGAGGTGGAGCCAATCCCAAAAAATGCGTCC
CAGTCCGGATTGCAGTCTGCAACTCGACTGCATGAAGTTGGAATCGCTAGT
AATTCGAGATCAGCATGCTCGGGTGAATGCGTTCCCGGGCCTTGTACACAC
CGCCCGTCACACCACGAAAGTCGGTTTACCCGATACCGGTGAGCCAACCG
CAAGGAGGCAGCCGTCTACGTAGACCCGG

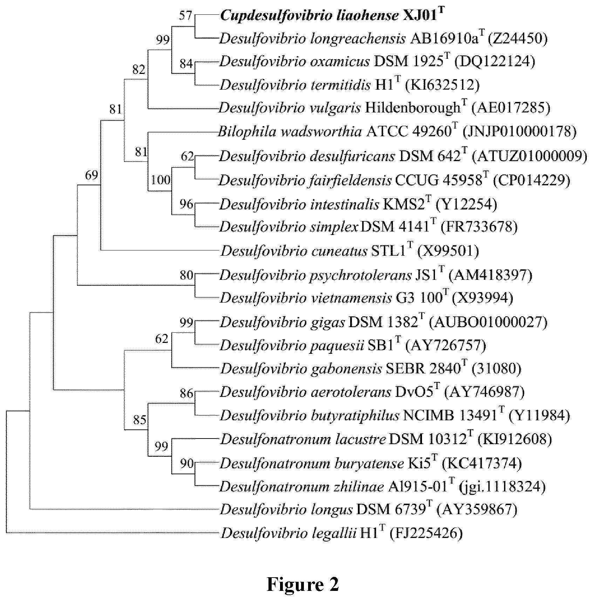

Homology alignments were conducted via EzTaxon-e (http://eztaxon-e.ezbiocloud.net/) and a phylogenetic tree for the screened strain and similar strains was constructed by the Neighbor-Joining method in the software MEGA5.0 (Figure 2). It was found by analysis that XJ01^{T} has the highest similarity with *Desulfovibrio longreachensis* AB16910a^{T} (97.89%), followed by *Desulfovibrio termitidis* DSM 5308^{T} (97.37%), *Desulfovibrio oxamicus* NCIMB 9442^{T} (97.30%) and *Desulfovibrio vulgaris* Hildenborough^{T} (93.77%).

In summary, the strain XJ01 purified according to the present disclosure appeared to be a new species in *Desulfovibrionales,* and was deposited in China General Microbiological Culture Collection Center (CGMCC) on July 20, 2018 (Address: Institute of Microbiology Chinese Academy of Sciences, Building 3, Yard No.1 West Beichen Road, Chaoyang District, Beijing, 100101) with the deposit number CGMCC No. 16138 and the taxonomic name *Cupidesulfovibrio* sp.

### Example 2: Reducing ability of the sulfate-reducing bacterial strain Cupidesulfovibrio sp. XJ01 under different initial concentrations of sulfate

Sodium lactate was used as the carbon source to investigate the effect of different SO₄²⁻ concentrations on the sulfate reduction by the strain XJ01. The enrichment medium was modified as follows: ferrous chloride was used instead of ammonium ferrous sulfate (ferrous chloride served to provide iron ions, and magnesium sulfate was used to provide all sulfate anions, for easy calculation).

The strain XJ01 was inoculated at 10% (v/v) into media with an initial SO₄²⁻ concentration of 0.5, 1, 10, 20, 30, 40, and 50 g/L, and the SO₄²⁻ concentration of each medium was measured after culture for 1, 2, 3, 4, and 5 days under optimal culturing conditions. The SO₄²⁻ concentration in the bacterial culture was determined by turbidimetry with the USEPA SulfaVer4 reagent.

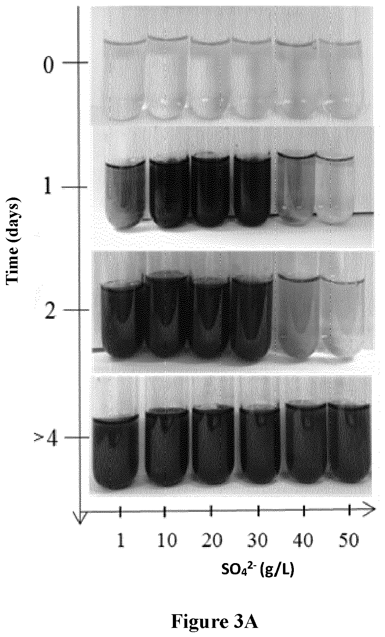

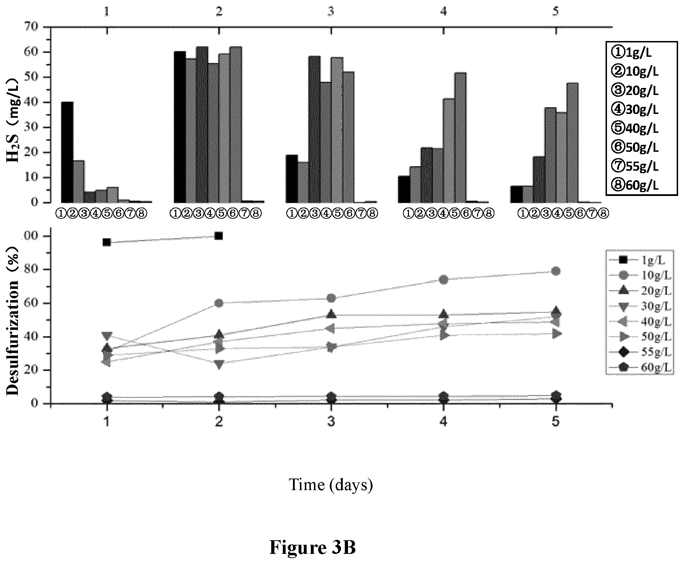

The results can be seen in Figure 3A and Figure 3B. The results of this example show that:
(1) at [SO₄²⁻]=0.5 g/L, the strain can achieve a sulfate reduction of 100% in 1 day;
(2) at [SO₄²⁻]=1 g/L, a sulfate reduction over 90% in 1 day and up to 100% in 2 days;
(3) at [SO₄²⁻]=10 g/L, a sulfate reduction of up to 60% in 2 days;
(4) at [SO₄²⁻]=20 g/L, a sulfate reduction of up to 44% in 3 days;
(5) at [SO₄²⁻]=30 g/L, a sulfate reduction of up to 37% in 5 days;
(6) at [SO₄²⁻]=40 g/L, a sulfate reduction of up to 25% in 5 days;
(7) at [SO₄²⁻]=50 g/L, a sulfate reduction of up to 13% in 5 days;
(8) at [SO₄²⁻]>50 g/L, the strain no longer grew.

Thus it can be seen that the strain has an extraordinary sulfate-reducing ability.

## Claims

1. A sulfate-reducing bacterium (*Cupidesulfovibrio* sp.) deposited with the deposit number CGMCC No. 16138.

2. A sulfate-reducing bacterium (*Cupidesulfovibrio* sp.) having a 16S rDNA sequence represented by SEQ ID No. 1.

3. A sulfate-reducing bacterial preparation, which is a solid or liquid bacterial preparation comprising the sulfate-reducing bacterium of claim 1 or 2.

4. A method for culturing the sulfate-reducing bacterium of claim 1 or 2 or the sulfate-reducing bacterial preparation of claim 3, the method comprising: culturing the sulfate-reducing bacterium of claim 1 or 2 or the sulfate-reducing bacterial preparation of claim 3 in a fermentation medium.

5. The method of claim 4, wherein the [SO₄²⁻] in the medium is less than or equal to 50 g/L, preferably 1 g/L to 50 g/L.

6. The method of claim 4, wherein the [SO₄²⁻] in the medium [SO₄²⁻] is 10 g/L.

7. The method of claim 4, wherein the culturing conditions are: a pH of 6 to 9, and a temperature of 20 to 45°C.

8. Metabolites of a sulfate-reducing bacterium, which are produced according to the method of any one of claims 4 to 7.

9. Use of the sulfate-reducing bacterium of claim 1 or 2, the sulfate-reducing bacterial preparation of claim 3, or the metabolites of claim 8, in sulfate reduction.

10. The use of claim 9, comprising using the sulfate-reducing bacterium to treat a sulfate-containing waste liquid.

11. The use of claim 10, wherein the [SO₄²⁻] in the waste liquid is less than or equal to 50 g/L.

12. The use of claim 10, wherein the [SO₄²⁻] in the waste liquid is 0.5 g/L to 50 g/L.

13. The use of claim 10, wherein the [SO₄²⁻] in the waste liquid is 1 g/L to 10 g/L.
